# FASCICULE DE BREVET EUROPEEN

(11) **EP 0 673 650 B1**
(45) Date de publication et mention de la délivrance du brevet: **01.08.2001**
(21) Numéro de dépôt: 95400521.1
(22) Date de dépôt: 13.03.1995
(51) Int. Cl.: A61K 31/505, A61K 9/28, A61K 9/50

(54) **Formes galéniques à libération prolongée du chlorhydrate d'alfuzosine**
Pharmazeutische Darreichungsformen mit verzögerter Wirkstoffabgabe enthaltend Alfuzosinhydrochlorid
Pharmaceutical sustained-release dosage forms containing alfuzosine hydrochloride

(30) Priorité: 21.03.1994 FR 9403257
(43) Date de publication de la demande: 27.09.1995
(73) Titulaire: SANOFI-SYNTHELABO, 75013 Paris (FR)
(72) Inventeur: Andrieu, Véronique, F-92100 Boulogne Billancourt (FR); Montel, Jean, F-78400 Chatou (FR); Wick, Alexander, F-78860 Saint Nom la Breteche (FR)
(74) Mandataire: Thouret-Lemaitre, Elisabeth

(56) Documents cités:
- EP-A- 0 204 597
- US-A- 4 315 007

## Description

La présente invention a pour objet des formes pharmaceutiques à libération ciblée du chlorhydrate d'alfuzosine et leur application à la préparation de formes galéniques à libération prolongée, utilisables pour une administration une fois par jour.

Le chlorhydrate d'alfuzosine, utilisé par voie orale dans le traitement symptomatique de l'hypertrophie bénigne de la prostate, présente une absorption dont l'intensité décroît le long du tractus digestif, l'absorption étant faible dans l'iléon et le colon.
Sous une forme orale classique, ce composé doit donc être administré plusieurs fois par jour.

Pour le chlorhydrate d'alfuzosine, une forme orale à libération prolongée permettant d'obtenir une concentration plasmatique constante et suffisante sur 24 heures, doit assurer sa libération et son absorption principalement dans les parties basses du tube digestif.

Les nouvelles formes galéniques selon la présente invention permettent de cibler la libération du chlorhydrate d'alfuzosine dans le tube digestif et s'appliquent à une administration orale une fois par jour, tout en apportant une grande sécurité vis-à-vis de toute libération trop rapide.

Les formes pharmaceutiques selon l'invention comportent un noyau : comprimé à libération immédiate ou à libération prolongée ou micrograins à libération immédiate du chlorhydrate d'alfuzosine, enrobé d'une membrane dont la nature et l'épaisseur permettent de contrôler la libération du principe actif en fonction du pH et du temps.

Les comprimés à libération immédiate ou prolongée du principe actif, dont toutes les dimensions sont inférieures à 10 mm, contiennent 3 à 10 % en poids de chlorhydrate d'alfuzosine.

Les premiers sont préparés par granulation aqueuse à partir du principe actif et d'excipients tels que lactose, cellulose microcristalline, polyvinylpyrrolidone, carboxyméthylamidon sodique et stéarate de magnésium, les seconds sont préparés par granulation aqueuse ou fusion, par association du principe actif à une matrice lipidique composée par exemple de cellulose microcristalline, de phosphate dicalcique dihydraté, d'huile de ricin hydrogénée, de polyvinylpyrrolidone et de stéarate de magnésium.

Les micrograins à libération immédiate du principe actif contiennent 3 à 15 % en poids de chlorhydrate d'alfuzosine et ont une taille comprise entre 0,50 et 1,25 mm. Ils sont préparés par granulation aqueuse à partir du principe actif, de mannitol et de polyvinylpyrrolidone.

Les comprimés et les micrograins sont ensuite entourés d'un film d'enrobage par pulvérisation d'une solution d'enrobage dans un appareil à lit d'air fluidisé ou tout autre dispositif satisfaisant.

L'enrobage contient un polymère à dissolution pH-dépendante, par exemple l'Eudragit S® (copolymère d'acide méthacrylique) qui permet d'obtenir un film d'enrobage se dissolvant à un pH supérieur à 7, assurant ainsi une libération colonique du principe actif.
L'épaisseur du film d'enrobage permet de moduler le temps de latence de libération du principe actif à pH = 7.

Dans le cas des micrograins, l'enrobage peut comprendre, en plus du polymère à dissolution pH-dépendante, un polymère imperméable. Il peut être constitué, par exemple, d'une association d'Eudragit S® et d'éthylcellulose, permettant de moduler la vitesse de libération du principe actif et assurant, comme dans le cas précédent, une libération du principe actif pH- et temps-dépendante par la présence du polymère soluble à un certain pH et par l'épaisseur du film d'enrobage.

Les formes pharmaceutiques selon l'invention peuvent contenir de 3 à 20 mg de chlorhydrate d'alfuzosine.

Elles peuvent être utilisées pour la préparation de formes galéniques à libération prolongée du chlorhydrate d'alfuzosine pour une administration quotidienne unique.

Ces formes galéniques peuvent comporter une ou plusieurs formes pharmaceutiques à noyaux enrobés, telles que définies précédemment, et éventuellement une ou plusieurs autres formes pharmaceutiques à noyaux enrobés ou pas Le mélange de ces différentes formes permet de moduler la libération du principe actif tout le long du tube digestif.

Les exemples suivants illustrent l'invention :

### Exemple 1 : comprimés pour une vectorisation colonique.

| comprimés | % (en poids) |
|---|---|
| chlorhydrate d'alfuzosine | 3,3 |
| lactose | 69,4 |
| cellulose microcristalline | 17,8 |
| polyvinylpyrrolidone | 5,0 |
| carboxyméthylamidon sodique | 4,0 |
| stéarate de magnésium | 0,5 |

| enrobage | |
|---|---|
| copolymère d'acide méthacrylique | 75,7 |
| monoglycérides diacétylés | 7,5 |
| talc | 16,8 |

### Exemple 2 : micrograins pour une vectorisation colonique.

| micrograins | % (en poids) |
|---|---|
| chlorhydrate d'alfuzosine | 7,0 |
| mannitol | 32,0 |
| cellulose microcristalline | 56,0 |
| polyvinylpyrrolidone | 5,0 |

| enrobage | |
|---|---|
| copolymère d'acide méthacrylique | 65,0 |
| éthylcellulose | 35,0 |
| monoglycérides diacétylés | 9,0 |

### Exemple 3 : comprimés dans une gélule pour une administration orale une fois par jour.

| comprimé n° 1 | % (en poids) |
|---|---|
| chlorhydrate d'alfuzosine | 3,3 |
| cellulose microcristalline | 30,0 |
| phosphate dicalcique dihydraté | 42,7 |
| huile de ricin hydrogénée | 18,0 |
| polyvinylpyrrolidone | 5,0 |
| stéarate de magnésium | 1,0 |

| comprimé n° 2 | |
|---|---|
| chlorhydrate d'alfuzosine | 3,3 |
| lactose | 69,4 |
| cellulose microcristalline | 17,8 |
| polyvinylpyrrolidone | 5,0 |
| carboxyméthylamidon sodique | 4,0 |
| stéarate de magnésium | 0,5 |

| enrobage du comprimé n° 2 | |
|---|---|
| copolymère d'acide méthacrylique | 75,7 |
| monoglycérides diacétylés | 7,5 |
| talc | 16,8 |

Des tests de dissolution de ces différentes formes pharmaceutiques ont été réalisés à pH = 2 et à pH = 7. Les résultats suivants ont été obtenus :

Formulation selon l'exemple 1, l'enrobage représentant 11 % du poids du comprimé et ayant une épaisseur de 100 *µ*m.

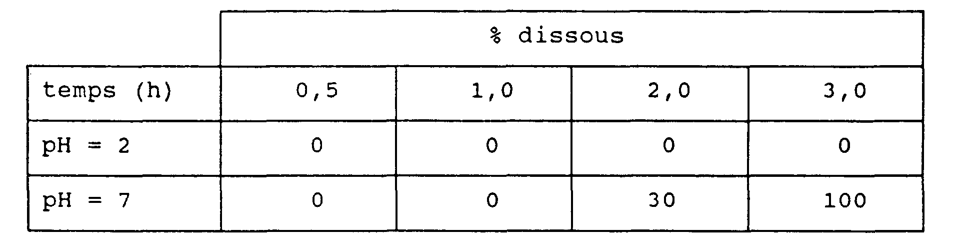

Cette formulation pour laquelle la libération du chlorhydrate d'alfuzosine est nulle à pH acide et totale en 3 heures à pH = 7, avec un temps de latence de 1 heure, permet d'assurer la libération du principe actif dans le colon.
En faisant varier l'épaisseur du film d'enrobage, on peut moduler le temps de latence de libération du principe actif à pH = 7.

Formulation selon l'exemple 2, l'enrobage représentant 14 % du poids des micrograins.

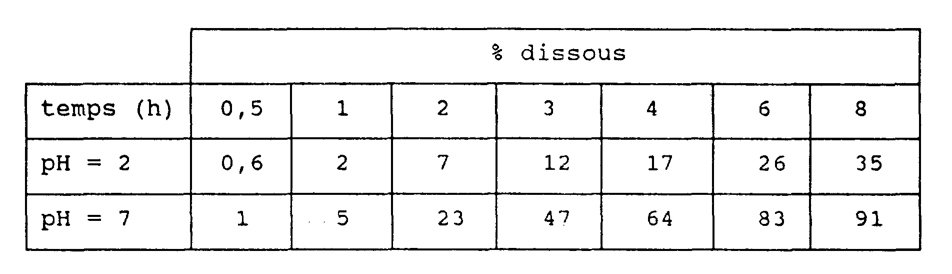

Cette formulation pour laquelle la libération du chlorhydrate d'alfuzosine varie en fonction du pH, permet d'assurer une vitesse de libération du principe actif variant tout le long du tube digestif.

Formulation selon l'exemple 3, l'enrobage du comprimé n° 2 représentant 11 % du poids de ce comprimé.

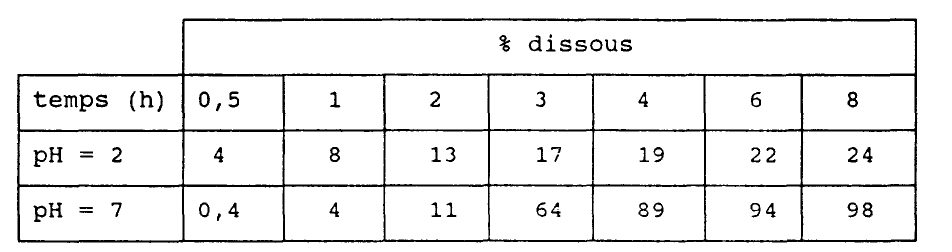

Cette formulation qui associe deux types de comprimés dans la même gélule assure une libération du chlorhydrate d'alfuzosine variable en fonction du pH et donc une vitesse de libération du principe actif variable tout le long du tube digestif.
En augmentant le nombre de comprimés à vectorisation colonique dans la gélule, on augmente la quantité de principe actif libéré dans le colon.

La cinétique plasmatique de formes pharmaceutiques selon l'invention a également été étudiée.

La cinétique plasmatique de comprimés à vectorisation colonique selon la formulation de l'exemple 1 a été déterminée chez douze volontaires sains après une administration unique par voie orale.
Les résultats obtenus ont montré que le comprimé de chlorhydrate d'alfuzosine arrive en moyenne au bout de 5 heures dans le colon et que le principe actif est libéré en un temps maximum de 11 heures. Le temps de demi-vie apparent de cette formulation est de 9 heures.

D'autre part, la cinétique plasmatique de gélules contenant des comprimés de chlorhydrate d'alfuzosine selon la formulation de l'exemple 3 a été étudiée.
Les résultats obtenus avec trois types de gélules contenant des proportions différentes des deux types de comprimés sont indiqués dans le schéma 1.
La courbe indiquée par 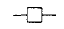 représente la cinétique plasmatique d'une gélule contenant 1 comprimé n° 1 non enrobé contenant 3 mg de chlorhydrate d'alfuzosine et 3 comprimés n° 2 enrobés contenant chacun 3 mg de chlorhydrate d'alfuzosine.
La courbe indiquée par 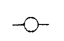 représente la cinétique plasmatique d'une gélule contenant 1 comprimé n° 1 non enrobé contenant 5 mg de chlorhydrate d'alfuzosine et 2 comprimés n° 2 enrobés de dosage identique à celui des comprimés n° 2 du cas précédent.
La courbe indiquée par 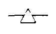 représente la cinétique plasmatique d'une gélule contenant 1 comprimé n° 1 non enrobé contenant 3 mg de chlorhydrate d'alfuzosine et 2 comprimés n° 2 enrobés de dosage identique à celui des comprimés n° 2 des cas précédents.

Les résultats montrent que cette formulation, qui permet de moduler la libération du chlorhydrate d'alfuzosine tout le long du tube digestif et de modifier ainsi la cinétique plasmatique, convient à une administration une fois par jour en apportant, grâce à la combinaison de dépendance au temps et au pH, une grande sécurité pour éviter toute libération plus rapide.

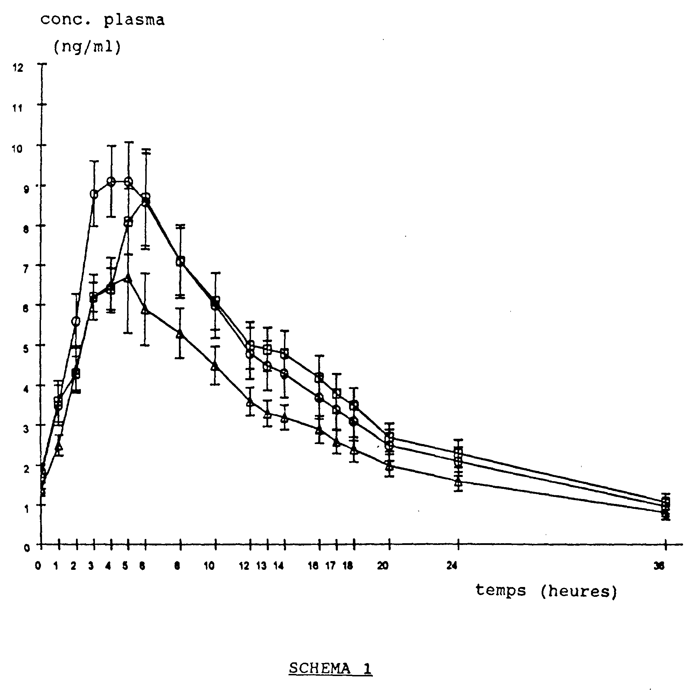

## Revendications

1. Forme pharmaceutique à libération ciblée du chlorhydrate d'alfuzosine, caractérisée en ce qu'elle comporte un noyau comprenant le principe actif, recouvert d'un enrobage contenant un polymère à dissolution pH-dépendante, ce polymère à dissolution pH-dépendante se dissolvant à un pH supérieur ou égal à 7.

2. Forme pharmaceutique selon la revendication 1, caractérisée en ce que le noyau est un comprimé constitué du principe actif et d'excipients.

3. Forme pharmaceutique selon la revendication 2, caractérisée en ce que le noyau est un comprimé constitué d'une matrice contenant le principe actif.

4. Forme pharmaceutique selon la revendication 1, caractérisée en ce que le noyau est constitué de micrograins.

5. Forme pharmaceutique selon l'une quelconque des revendications 1 à 4, caractérisée en ce que le polymère à dissolution pH-dépendante est un copolymère d'acide méthacrylique dont la nature et l'épaisseur permettent d'assurer une libération du principe actif dépendante du temps et du pH.

6. Forme pharmaceutique selon la revendication 4, caractérisée en ce que l'enrobage contient de l'éthylcellulose.

7. Forme pharmaceutique selon l'une quelconque des revendications 1 à 6, caractérisée en ce qu'elle contient 3 à 20 mg de chlorhydrate d'alfuzosine.

8. Forme galénique à libération prolongée du chlorhydrate d'alfuzosine, caractérisée en ce qu'elle comporte une ou plusieurs formes à noyaux enrobés selon l'une quelconque des revendications 1 à 7 et, éventuellement, une ou plusieurs autres formes à noyaux enrobés ou pas.

9. Forme galénique selon la revendication 8, caractérisée en ce que les différentes formes sont contenues dans une gélule.

## Patentansprüche

1. Pharmazeutische Zubereitung mit gezielter Freisetzung von Alfuzosin-Hydrochlorid, dadurch gekennzeichnet, daß sie einen den Wirkstoff enthaltenden Kern umfaßt, der mit einer Umhüllung bedeckt ist, die ein Polymer mit pH-abhängiger Auflösung enthält, welches Polymer mit pH-abhängiger Auflösung sich bei einem pH-Wert von gleich oder größer 7 auflöst.

2. Pharmazeutische Zubereitung nach Anspruch 1, dadurch gekennzeichnet, daß der Kern in Form einer Tablette vorliegt, die aus dem Wirkstoff und Hilfsstoffen gebildet ist.

3. Pharmazeutische Zubereitung nach Anspruch 2, dadurch gekennzeichnet, daß der Kern eine Tablette ist, die aus einer den Wirkstoff enthaltenden Matrix gebildet ist.

4. Pharmazeutische Zubereitung nach Anspruch 1, dadurch gekennzeichnet, daß der Kern aus Mikrokörnchen gebildet ist.

5. Pharmazeutische Zubereitung nach irgendeinem der Ansprüche 1 bis 4, dadurch gekennzeichnet, daß das Polymer mit pH-abhängiger Auflösung ein Methacrylsäure-Copolymer ist, dessen Art und Dicke es ermöglicht, eine Freisetzung des Wirkstoffs in Abhängigkeit von der Zeit und dem pH-Wert sicherzustellen.

6. Pharmazeutische Zubereitung nach Anspruch 4, dadurch gekennzeichnet, daß die Umhüllung Ethylcellulose enthält.

7. Pharmazeutische Zubereitung nach irgendeinem der Ansprüche 1 bis 6, dadurch gekennzeichnet, daß sie 3 bis 20 mg Alfuzosin-Hydrochlorid enthält.

8. Galenische Form mit verzögerter Freisetzung von Alfuzosin-Hydrochlorid, dadurch gekennzeichnet, daß sie einen oder mehrere umhüllte Kerne nach irgendeinem der Ansprüche 1 bis 7 und gegebenenfalls einen oder mehrere weitere gegebenenfalls umhüllte Kerne umfaßt.

9. Galenische Form nach Anspruch 8, dadurch gekennzeichnet, daß die verschiedenen Formen in einer Gelkapsel enthalten sind.

## Claims

1. Targeted-release pharmaceutical form of alfuzosin hydrochloride, characterized in that it contains a core comprising the active principle, coated with a coating containing a polymer whose dissolution is pH-dependent, this polymer with pH-dependent dissolution dissolving at a pH which is greater than or equal to 7.

2. Pharmaceutical form according to Claim 1, characterized in that the core is a tablet consisting of the active principle and excipients.

3. Pharmaceutical form according to Claim 2, characterized in that the core is a tablet consisting of a matrix containing the active principle.

4. Pharmaceutical form according to Claim 1, characterized in that the core consists of microparticles.

5. Pharmaceutical form according to any one of Claims 1 to 4, characterized in that the polymer whose dissolution is pH-dependent is a methacrylic acid copolymer whose nature and thickness enable a time- and pH-dependent release of the active principle to be effected.

6. Pharmaceutical form according to Claim 4, characterized in that the coating contains ethylcellulose.

7. Pharmaceutical form according to any one of Claims 1 to 6, characterized in that it contains 3 to 20 mg of alfuzosin hydrochloride.

8. Sustained-release dosage form of alfuzosin hydrochloride, characterized in that it contains one or more forms having coated cores according to any one of Claims 1 to 7 and, optionally, one or more other forms having coated or uncoated cores.

9. Dosage form according to Claim 8, characterized in that the different forms are contained in a hard gelatin capsule.
